# EUROPEAN PATENT SPECIFICATION

(11) **EP 0 930 880 B1**
(45) Date of publication and mention of the grant of the patent: **24.09.2003**
(21) Application number: 97945018.6
(22) Date of filing: 15.10.1997
(51) Int. Cl.: A61K 31/34, A61K 31/616, A61K 9/20, A61P 9/10

(54) **PHARMACEUTICAL COMPOSITION COMPRISING A COMBINATION OF ISOSORBIDE NITRATE WITH ACETYLSALICYLIC ACID**
PHARMAZEUTISCHE ZUBEREITUNGEN ENTHALTEND EINE KOMBINATION AUS ISOSORBID-NITRAT UND ACETYLSALICYLSAEURE
COMPOSITION PHARMACEUTIQUE COMPRENANT UNE COMBINAISON DE NITRATE D'ISOSORBIDE ET D'ACIDE ACETYLSALICYLIQUE

(30) Priority: 18.10.1996 GB 9621749; 26.06.1997 GB 9713549
(43) Date of publication of application: 28.07.1999
(73) Proprietor: Euro-Celtique S.A., Luxemburg (LU)
(72) Inventor: MILLER, Ronald Brown, Résidence du Soleil, CH-4051 Basle (CH); LESLIE, Stewart, Thomas, Cambridge CB2 2RA (GB); MALKOWSKA, Sandra Therese Antoinette, Wilburton, Ely CB6 3RT (GB); PRATER, Derek Allan, Milton, Cambridge CB4 4YS (GB)
(74) Representative: Ruffles, Graham Keith
(86) International application number: GB9702849
(87) International publication number: WO98017271

(56) References cited:
- EP-A- 0 654 263
- EP-A- 0 676 204
- WO-A-94/03421
- V.G.ATHYROS ET AL.: "COMBINED TREATMENT WITH PRAVASTATIN AND GEMFIBROZIL IN PATIENTS WITH REFRACTORY FAMILIAL COMBINED HYPERLIPIDAEMIA. A CLINICAL STUDY" DRUG INVEST., vol. 7, no. 3, 1994, NEW ZEALAND, pages 134-142, XP002054179
- HEYDT-LIMAN, A. ET AL.: "PHARMACOKINETICS OF ISOSORBIDE DINITRATE WHEN COADMINISTERED WITH ACETYLSALICYLIC ACID" EUR. J. PHARMACOL., vol. 183, no. 6, 1990, NETHERLANDS, page 2395 XP002054180
- WAI LIN NG, ET AL.: "SURGERY FOR LEFT MAIN SPASM. IS IT INDICATED?" INT. J. CARDIOL., vol. 54, no. 3, June 1996, IRELAND , pages 213-216, XP002054181
- DATABASE WPI Section Ch, Week 8633 Derwent Publications Ltd., London, GB; Class B02, AN 86-216093 XP002054182 & JP 61 148 115 A (TOA EIYO LTD) , 5 July 1986

## Description

This invention relates to a pharmaceutical composition, in particular a pharmaceutical composition for the treatment of vascular disease and/or its symptoms, such as angina pectoris and cardiovascular, cerebral vascular and peripheral vascular disease.

Isosorbide dinitrate and mononitrate are widely prescribed for the prophylactic treatment of angina. Among the marketed preparations containing these substances are IMDUR 60 mg tablets of Astra Pharmaceuticals Ltd, and ELANTAN LA 25 mg and 50 mg capsules sold by Schwarz Pharma Ltd, both of which are sustained release preparations containing isosorbide-5-mononitrate (I-5-MN).

Acetylsalicylic acid (Aspirin) or its salts, are sold in various forms for the treatment of cardiovascular disease, for instance, for the prevention of secondary myocardial infarcts (MI), and also as an antithrombotic in patients with unstable angina or cerebral transient ischaemic attacks. The recommended dosages vary from 75 mg to 150 mg of aspirin daily.

In European Journal of Clinical Pharmacology (1983) 25:779-782, Ray et al. describe the pharmacological interaction between nitroglycerine and aspirin in healthy subjects. They observed that when the subjects were pre-treated, either chronically or acutely with aspirin both the Cmax and AUC of nitroglycerine were increased, probably representing an increase in the amount of nitroglycerin absorbed. The increase in nitroglycerin concentration with aspirin led to changes in heart rate, and to a decrease in diastolic arterial pressure and various other parameters. The authors concluded that the results suggested a complex interaction between nitroglycerin and aspirin. A complex interaction between nitroglycerin and aspirin was also reported by Weber et al. in Journal of Cardiovascular Pharmacology 5: 874-877, 1983.

In European Heart Journal (1988) 9 (Supplement A), 45-49, De Caterina et al. reported on the ability of isosorbide dinitrate and the two main metabolites, isosorbide 2-mononitrate (I-2-MN) and I-5-MN to inhibit platelet aggregation and thromboxane B2 generation in vitro in response to threshold concentrations of ADP, adrenaline, collagen, thrombin and arachidonic acid. In the Discussion at page 48, right hand column, last seven lines they note that "contrary to aspirin, however, nitrates appear to also affect primary aggregation, which suggests different targets on platelet aggregation mechanisms. There are, therefore, possibilities that such effects are additive to those of other agents such as aspirin."

In European Heart Journal (1991) 12 (Supplement A), 2-4, Conti reviewed the therapeutic use of nitrates, as of 1990, and observed that "nitrates are beneficial because they combine coronary and non-coronary effects. They have proven clinical efficacy, relieving symptoms and documented evidence of ischaemia. They can be given to patients by multiple routes and can be easily combined with other anti-ischaemic drugs including aspirin, heparin, calcium antagonists and beta blockers." He noted that the additive effects of nitrates to aspirin, postulated by De Caterina et al., may be particularly beneficial in patients with unstable angina and myocardial infarction.

EP 0396425 (KV Pharmaceutical Company) describes, in Example 10, a formulation containing I-5-MN, in which the 1-5-MN is applied as a triturate on sugar spheres by means of a pharmaceutical glaze and a solution of povidone. After drying and removal of solvents a coating of talc is applied using polyvinyl chloride and pharmaceutical glaze and drying and removal of solvents must then be repeated. After preparing the beads in the above described manner, a controlled-release layer is applied comprising ethylcellulose and diethyl phthalate in an organic solvent system containing isopropanol and methylene chloride, as well as talc and calcium stearate. Further drying and solvent removal is then required.

European Patent Application Publication No. 0477061 describes a sustained release formulation containing I-5-MN in the form of a tablet containing a powder of I-5-MN of specified particle size homogeneously dispersed in a hydrophilic matrix based on at least one swellable excipient e.g. protein or cellulosic hydrocolloids such as alginates, or methylcellulose or hydroxypropylcellulose, and at least one diluent such as xanthan gum or microcrystalline cellulose.

European Patent Application Publication No. 0219161 describes sustained release I-5-MN tablets containing polyvinyl pyrollidone (PVP) which is stated to have the properties of acting as a binder to the components, including the matrix which imparts the retardant effect to the I-5-MN tablets, and also forms a solid stable solution with the I-5-MN. The tablets described in this document contain, in addition to the PVP, hydroxypropylmethylcellulose, hydroxypropyl cellulose and various other ingredients.

German Patent Specification No. 4313726 describes a solid oral dosage form having a pH independent release rate of the I-5-MN. The dosage form comprises inert sugar beads coated with a composition containing I-5-MN suspended in a mixture containing inter alia PVP and hydroxypropylmethylcellulose, to which a further coating is applied of an I-5-MN-free suspension containing inter alia PVP and hydroxypropylmethylcellulose. The resulting beads are then provided with a release controlling coating containing ethylcellulose, polymethacrylate and dibutylphthalate.

In PCT Patent Application PCT/IE93/00040, WO 9403421, there is proposed a combination formulation containing an amount of aspirin in normal release form and organic nitrate in immediate and/or controlled release form. The formulation is particularly described as a capsule containing a tablet comprising isosorbide mononitrate in a matrix of a release controlling material, together with acetylsalicylic acid in powder or granular form for normal release. The I-5-MN containing tablet is obtained by blending I-5-MN with calcium hydrogen phosphate and Eudragit NE 40D (an aqueous dispersion of a neutral copolymer based on ethyl acrylate and methyl methacrylate), wet granulating and then compression.

European patent application publication number 0654263 provides a process for the manufacture of particles which comprises mechanically working a mixture of a drug and a hydrophobic and/or hydrophilic fusible carrier in a high speed mixture so as to form agglomerates, breaking the agglomerates to give controlled release particles and optionally continuing the mechanical working with the optional addition of a low percentage of the carrier of diluent.

According to the present invention, there is provided a multiphase tablet, for the treatment of vascular disease, having a first phase which contains a therapeutically effective amount of isosorbide nitrate, preferably 1-5-MN in a controlled release matrix comprising a hydrophobic and/or lipophilic, non-swelling fusible carrier or diluent and a second phase which contains a therapeutic amount of acetylsalicylic acid or pharmaceutically acceptable salt thereof, there being no barrier layer between the first and second phases the second phase being adapted to rapidly disintegrate or being in normal release form and wherein the tablet when administered orally is capable of providing therapeutic blood levels of the isosorbide nitrate over a period of at least 12 hours, preferably over a period of 24 hours.

The multiphase tablet in accordance with the invention is preferably a bi-layer tablet.

Preferably a tablet according to the present invention contains in the first phase 20 mg to 120 mg of the isosorbide nitrate, preferably I-5-MN. In its most preferred form the tablet contains I-5-MN in the first phase in an amount of 40 mg to 120 mg, preferably 60 mg.

Suitable substances for use as hydrophobic and/or lipophilic non-swelling, fusible carrier or diluent materials are natural or synthetic waxes or oils, for example hydrogenated vegetable oil, hydrogenated castor oil, Beeswax, Carnauba wax, microcrystalline wax and glycerol monostearate, and suitably have melting points of from 35°C to 150°C, preferably 45°C to 90°C. The matrix may contain one or more of such materials.

The first phase may contain 40%-90% by weight of isosorbide nitrate preferably 1-5-MN and in particularly preferred embodiments may contain between 45% and 70% by weight of isosorbide nitrate preferably 1-5-MN. Preferably the controlled release matrix consists of, or substantially consists of, the hydrophobic and/or lipophilic, non-swelling fusible carrier or diluent which will be 10% to 60% by weight of the first Phase and will usually amount to over 30% by weight of the first phase.

By providing a second phase which rapidly disintegrates or is in normal release form, the surface of the first phase which is originally contacted by the second phase and therefore masked from the gastric fluid rapidly becomes exposed to that fluid, with the result that interference by the second phase in the dissolution of the isosorbide nitrate from the first phase is minimal or rendered insignificant. This ensures more consistent dissolution patterns and resulting blood plasma profiles.

A multiphase tablet according to the invention which is capable of providing a therapeutically effective blood level of isosorbide nitrate, preferably I-5-MN over at least 12 or 24 hours will, when tested according to the Ph. Eur Paddle method at 100 rpm in pH 4.5 buffer using tablet sinkers, and HPLC detection, preferably release the isosorbide nitrate, preferably I-5-MN at the following rate:-

| | |
|---|---|
| 1 hour | 15%-50% |
| 2 hours | 25%-65% |
| 4 hours | 40%-85% |
| 8 hours | 60%-100% |
| 12 hours | > 60% |

Suitably the tablets for dosing at intervals of at least 12 or 24 hours may demonstrate a release rate of isosorbide nitrate, preferably I-5-MN when tested as aforesaid, as follows:-

| | |
|---|---|
| 1 hour | 20%-40% |
| 2 hours | 33%-53% |
| 4 hours | 50%-70% |
| 8 hours | 67%-87% |
| 12 hours | 70%-100% |

Multiphase tablets in accordance with the invention suitable for at least 12 or 24 hourly dosing may also demonstrate the following release rate of isosorbide mononitrate, preferably I-5-MN when tested as aforesaid:-

| | |
|---|---|
| 1 hour | 15%-35% |
| 2 hours | 25%-45% |
| 4 hours | 40%-60% |
| 8 hours | 60%-80% |
| 12 hours | 70%-90% |

It has also been found that multiphase tablets which are suitable for at least 12 or 24 hourly dosing may, when tested as aforesaid, may have release rates of the isosorbide nitrate, preferably I-5-MN as follows:-

| | |
|---|---|
| 1 hour | 30%-50% |
| 2 hours | 45%-65% |
| 4 hours | 65%-85% |
| 8 hours | > 85% |
| 12 hours | > 90% |

The second phase may contain minor amounts of conventional excipients such as anti-tack agents, lubricants, disintegrants and compression aids such as talc, colloidal silica, natural or synthetic wax or oil and starch. These substances are preferably present, if at all, in a total amount corresponding to 0.5% to 20%, preferably 5% to 20%, most preferably 9% to 16% by weight of the acetylsalicylic acid or pharmaceutically acceptable salt thereof in this phase, the remainder of the phase preferably consisting of acetylsalicylic acid or pharmaceutically acceptable salt thereof.

A preferred second phase contains as excipients a compression aid, preferably starch, in an amount of about 8% to 13% by weight of the acetylsalicylic acid or pharmaceutically acceptable salt. Another preferred second phase contains, especially one where starch is used as a compression aid, contains a lubricating agent, preferably natural or synthetic wax or oil, preferably hydrogenated vegetable oil, in an amount of 0.5% to 6% e.g. 0.5% to 2.5% by weight of the acetylsalicylic acid or pharmaceutically acceptable salt thereof.

In preferred embodiments of tablets in accordance with the invention, when tested by Ph. Eur. Paddle Method operating at 50 rpm with pH 4.5 buffer as the dissolution medium, the second phase is substantially completely disintegrated or the acetylsalicylic acid or salt thereof completely dissolved within 45 minutes, preferably 25 minutes, most preferably 10 minutes.

We have found that the use of a minor amount of natural or synthetic wax or oil, in the second phase, provides a satisfactory lubrication for the tabletting process, in that the granulates are free flowing but advantageously no sticking in the tablet press is encountered, and at the same time the resulting phase of the tablet is nonetheless rapidly dissolving or disintegrating.

The natural or synthetic wax or oil in the second phase may be chosen from those mentioned above in relation to the isosorbide nitrate-containing first phase and is preferably hydrogenated vegetable oil.

When the second phase is rapidly disintegrating, the particles of acetylsalicylic acid or pharmaceutically acceptable salt thereof may be coated with a release rate modifying coating, for instance they may be coated with a composition containing enteric material such as shellac, cellulose derivatives such as cellulose acetate phthalate, hydroxypropyl methylcellulose phthalate or methacrylate polymers as well as ethyl cellulose.

In the multiphase tablets in accordance with the invention, however, it is not required to provide a barrier layer between the isosorbide-5-mononitrate-containing phase and the aspirin-containing phase, in contrast to the formulation described in e.g. PCT/IE93/00040.

The multiphase tablets in accordance with the invention may be manufactured by processes which do not involve the presence of organic solvents or water and are prepared by compressing granules, agglomerates, pellets or multiparticulates obtained as generally described in European Patent Application Publication No. 0654263, International Patent Application Publications No. WO 96/14059 and WO 93/18753 and United Kingdom Patent Specification No. 1513166.

It is known that chronic dosing of patients with certain non-steroidal anti-inflammatory drugs, including especially acetyl salicylic acid (aspirin) will often lead to damage to the gastric mucosa, causing erosive gastritis and gastric haemorrhage. Symptoms are meleana, heartburn, nausea and vomiting. Such damage is widely recognised in the case of dosing at, say 1000 mg aspirin per day with blood levels of 100 µg/ml of salicylate and there is also evidence that at much lower levels of dosing e.g. below 150 mg aspirin per day similar side effects will occur.

There are number of published reports of the use of nitric oxide (NO) in reducing the severity of gastric mucosal lesions.

There have been no reports or suggestions that dosing with a controlled release formulation of isosorbide nitrate, preferably isosorbide mononitrate, could achieve the prevention, elimination, or reduction in damage to the gastric and intestinal mucosa resulting from repeated dosing with an instant releasing formulation of low dose aspirin e.g. 150 mg or less of aspirin per day.

It is surprising that a formulation of isosorbide nitrate, which releases the active ingredient in a controlled manner such that a significant portion thereof is absorbed in the gastrointestinal tract can effectively counteract the damaging side effects of aspirin which is released and absorbed substantially but not entirely in the stomach.

In the preparations according to the present invention the isosorbide nitrate advantageously performs a dual role in acting both as an anti anginal agent and an agent which prevents or reduces the damaging side effects of the accompanying aspirin on the gastric mucosa.

Tablets containing isosorbide nitrate, preferably 1-5-MN in accordance with the invention may be manufactured by a process which comprises:-
(a) mechanically working in a high-shear mixer, a mixture of the isosorbide nitrate in particulate form and a particulate, hydrophobic and/or lipophilic, non-swellable fusible carrier or diluent having a melting point from 35 to 150°C and optionally a release control component comprising a water soluble fusible material or a particulate, soluble or insoluble organic or inorganic material, at a speed and energy input which allows the carrier or diluent to melt or soften, whereby it forms granules;
(b) breaking down or classifying the larger granules to give controlled release granules;
(c) compressing the granules with the optional use of lubricants e.g. talc, magnesium stearate, hydrogenated vegetable oil or other compression aids to form a tablet.

Alternatively, the tablets may be manufactured by a process which comprises pelletising a mixture containing the isosorbide nitrate in finely divided form and a particulate binder consisting of one or more water-insoluble wax-like binder substances with a melting point above 40°C, wherein the pelletisation step is performed by mechanically working the mixture in a high shear mixer, under the input of a sufficient amount of energy for the binder to melt and pelletisation to take place. The resulting pellets are then compressed to form tablets, optionally after mixing with tabletting aids such as lubricants e.g. talc and magnesium stearate.

The tablets may also be manufactured by a process which comprises:-
(a) mechanically working in a high-shear mixer, a mixture of particulate isosorbide nitrate and a particulate, hydrophobic and/or lipophilic fusible carrier or diluent having a melting point from 35 to 150°C and optionally a release control component comprising a water-soluble fusible material or particulate, soluble or insoluble organic or inorganic material, at a speed and energy input which allows the carrier or diluent to melt or soften whereby it forms granules; and
(b) extruding the resulting material and forming the extruded material into multiparticulates e.g. by cutting.

The resulting multiparticulates are then compressed to form tablets, optionally after mixing with tabletting aids such as lubricants.

Stage (a) of the processes mentioned above can be carried out in conventional jacketed high-shear mixers with a standard stainless steel interior, e.g. a Collette Gral 10, Vactron 75 or equivalent mixer. The mixture is processed until a bed temperature above 40°C is achieved and the resulting mixture acquires a cohesive granular texture. Such material, in the case of the embodiments described below, has the appearance of granules which, upon cooling below 40°C have structural integrity and resistance to crushing between the fingers. At this stage the granules are of an irregular size, shape and appearance.

The granules are preferably allowed to cool. The temperature to which they cool is not critical and a temperature in the range room temperature to 41°C may be conveniently used.

In the first process described above the granules are broken down by any suitable means which will comminute oversize granules and produce a mixture of powder and small, irregular shaped particles. It is currently preferred to carry out the classification using a sieve or a cone mill with an appropriate sized screen. We have found that if too small a screen size is used in the aforementioned apparatus, the granules, melting under the action of the impeller, will clog the screen and prevent further throughput of mixture and thus reduce yield.

Small amounts of release control components may optionally be used, such as a water soluble, fusible material e.g. a PEG of appropriate molecular weight; suitable particulate inorganic and organic materials, for example dicalcium phosphate, calcium sulphate, talc, colloidal anhydrous silica, and lactose, poloxamers and microcrystalline cellulose.

To produce tablets in accordance with the invention, particles produced as described above may be mixed or blended with the desired tabletting excipient(s) e.g. talc, magnesium stearate or hydrogenated vegetable oil, if any, using conventional procedures e.g. using a Y-cone or bin-blender and the resulting mixture compressed according to conventional tabletting procedure using a suitably sized tabletting tooling. Tablets can be produced using conventional tabletting machines; the embodiments described below were produced on a standard single punch F3 Manesty machine or Kilian RLE15 rotary tablet machine or BB3B rotary tablet machine.

The present pharmaceutical composition, when in the form of a multiphase tablet, may be prepared by compressing isosorbide nitrate-containing particles as described above and particles comprising acetylsalicylic acid (or pharmaceutically acceptable salt thereof) and optionally excipients as described above in a conventional manner, to give a multiphase tablet e.g. a bi-layer tablet.

The material for compression to form the acetylsalicylic acid or pharmaceutically acceptable salt-containing phase can be prepared by simply blending in a suitable blender the required ingredients in particulate form. When the ingredients are the active material and natural or synthetic wax or oil is used as lubricant and starch as a compression aid it is sufficient to carry out blending and it is not necessary to granulate the material.

### Example 1

Tablets were made according to the following procedure:-
Granules containing I-5-MN were prepared following the procedure listed below and having the following ingredients:-

| Ingredients | | mg per tablet |
|---|---|---|
| (a) | Isosorbide-5-mononitrate | 60.0 |
| (b) | Hydrogenated vegetable oil | 49.0 |
| (c) | Magnesium stearate Ph. Eur. | 3.48 |
| (d) | Talc Ph. Eur. | 3.48 |

Ingredients (a) and (b) (total batch weight of 18 kg) were placed in the bowl of a 75 litre capacity Vactron mixer equipped with variable speed mixing, granulating blades, a heated jacket to the mixing bowl and a microwave generator.

The ingredients were mixed at about 130 rpm with the mixer blade and using the granulating blade whilst applying microwave energy and heating via the heating jacket to soften the hydrogenated vegetable oil until the contents of the bowl form granules.

The granules were then classified through a sieve and/or cone mill to obtain controlled release granules.

The granules were then blended with ingredients (c) and (d) using a Y cone or a bin blender.

A powder mixture was obtained by blending acetylsalicylic acid, hydrogenated vegetable oil, starch and talc, all in particulate form, in the following proportions:-

| | Ingredients | mg per tablet |
|---|---|---|
| (i) | Acetylsalicylic acid | 75.0 |
| (ii) | Starch | 8.33 |
| (iii) | Hydrogenated vegetable oil | 0.868 |
| (iv) | Talc | 2.60 |

The granules blended with (c) and (d) and the powder mixture were then compressed to give a I-5-MN/acetylsalicylic acid bi-layer tablet using a 8.0 mm flat bevelled edged round tooling on a BB3B rotary tabletting machine.

### Example 2

### I-5-MN containing layer.

Following the procedure of Example 1 a bi-layer tablet was produced having the following ingredients

| | Ingredient | mg per tablet |
|---|---|---|
| (a) | Isosorbide-5-mononitrate | 60.0 |
| (b) | Hydrogenated vegetable oil BP | 49.0 |
| (c) | Magnesium stearate | 3.48 |
| (d) | Talc | 3.48 |

### II Acetylsalicylic acid-containing layer

| | | |
|---|---|---|
| (i) | Acetylsalicylic acid | 150 |
| (ii) | Starch | 16.6 |
| (iii) | Hydrogenated vegetable oil | 1.74 |
| (iv) | Talc | 5.20 |

### Example 3

| | Ingredients | mg per tablet |
|---|---|---|
| (a) | Isosorbide-5-mononitrate | 60.0 |
| (b) | Hydrogenated vegetable oil BP | 29.6 |
| (c) | Talc Ph. Eur. | 2.80 |
| (d) | Magnesium Stearate Ph. Eur. | 0.930 |

Particles containing the ingredients (a) and (b) which were in finely divided form were prepared by the steps of:-
(i) Placing ingredients (a) and (b) (total batch weight 0.7 kg) in the bowl of a 10 litre capacity Collette Gral Mixer (or equivalent) equipped with variable speed mixing, granulating blades and a heated jacket to the mixing bowl.
(ii) Mixing the ingredients at about 300 rpm whilst applying heating to soften the hydrogenated vegetable oil until the contents of the bowl are formed into granules.
(iii) Cooling the granules and classifying the material by passage through a Comic to obtain controlled release granules.

The granules were then blended with ingredients (c) and (d) using a Y-cone blender or a bin-blender. The blended mixtures were then each compressed on a 6.3 mm deep concave round tooling on a single punch F3 Manesty tabletting machine.

### Example 4

Following the procedure of Example 3, tablets having the following ingredients were produced:-

| | Ingredients | mg per tablet |
|---|---|---|
| (a) | Isosorbide-5-mononitrate | 60.00 |
| (b) | Hydrogenated vegetable oil BP | 40.00 |
| (c) | Talc Ph. Eur. | 3.13 |
| (d) | Magnesium Stearate Ph. Eur. | 1.04 |

The tablets were obtained by compression of the obtained blended mixtures on a 6.3 mm deep concave round tooling on a single punch F3 Manesty tabletting machine.

### Example 5

Following the procedure of Example 1 tablets having the following ingredients were produced:-

| | Ingredients | mg per tablet |
|---|---|---|
| (a) | Isosorbide-5-Mononitrate | 60.00 |
| (b) | Hydrogenated vegetable oil BP | 49.00 |
| (c) | Talc Ph. Eur | 3.41 |
| (d) | Magnesium Stearate Ph. Eur. | 1.14 |

### Reference Example 6

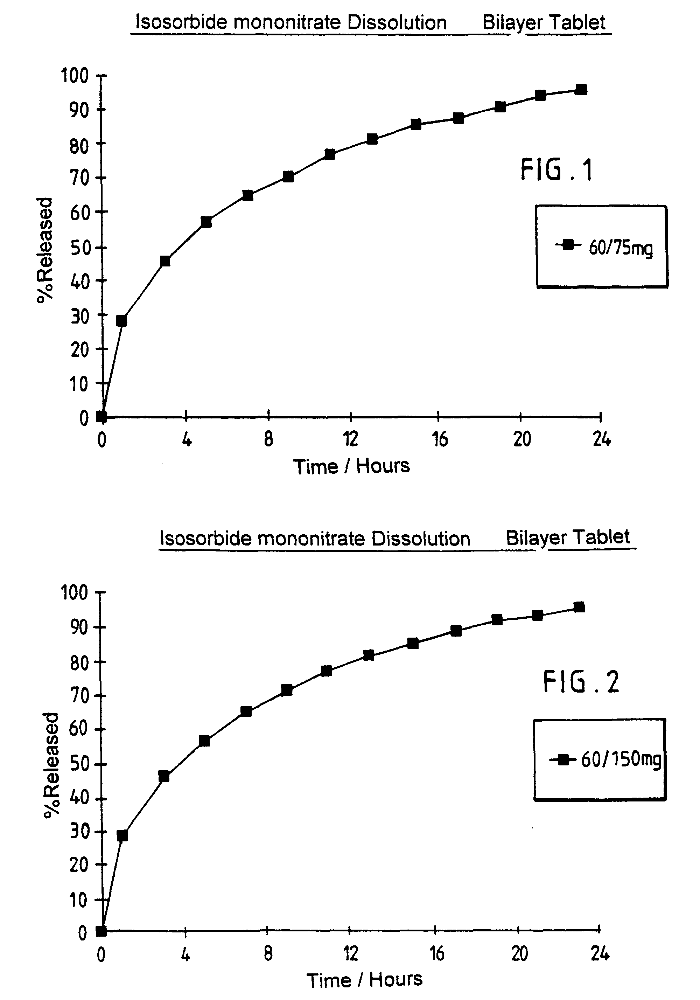

Tablets prepared according to Examples 1 and 2 were tested according to Ph. Eur. Paddle method in a buffered solution at pH. 4.5, with a sinker, at a paddle speed of 100 rpm and HPLC detection. The results obtained using the different tablets are shown respectively in the attached Figures 1 and 2

### Reference Example 7

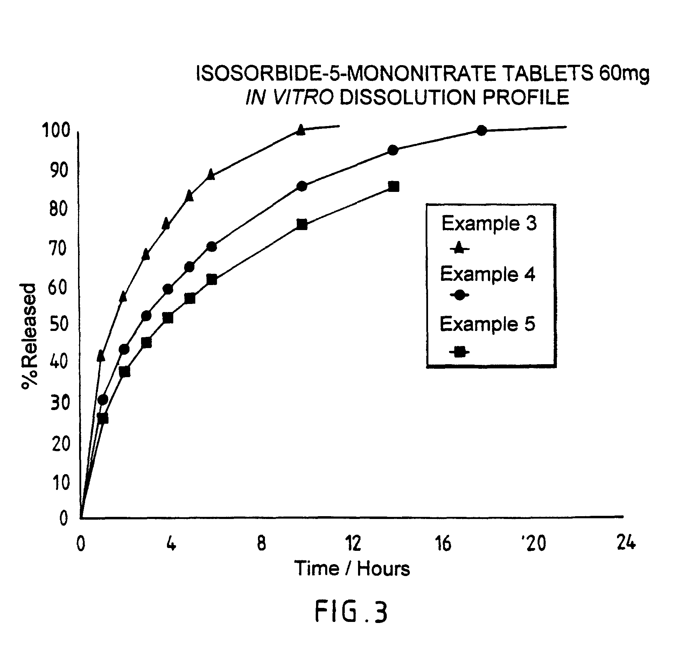

Tablets prepared according to Examples 3, 4 and 5 were tested according to Ph. Eur. Paddle method in a buffered solution at pH. 4.5, with a sinker, at a paddle speed of 100 rpm and HPLC detection. The results obtained using the different tablets are shown in the attached Figure 3.

### Example 8

Granules containing I-5-MN were prepared according to Examples 3, 4 or 5. These, and 75 mg or 150 mg acetylsalicylic acid in particulate form, were then compressed to give I-5-MN/acetylsalicylic acid bilayer tablets.

## Claims

1. A multiphase tablet, for the treatment of vascular disease having a first phase which contains a therapeutically effective amount of isosorbide nitrate, preferably isosorbide-5-mononitrate, in a controlled release matrix comprising a hydrophobic and/or lipophilic, non-swelling, fusible carrier or diluent, and a second phase which contains a therapeutically effective amount of acetylsalicylic acid or pharmaceutically acceptable salt thereof, there being no barrier layer between the first and second phases the second phase being adapted rapidly to disintegrate and/or being in normal release form, and wherein the tablet, when administered orally, is capable of providing therapeutic blood levels of the isosorbide nitrate over a period of at least 12 hours.

2. A multiphase tablet according to claim 1, wherein the tablet is capable of providing therapeutic blood levels of the isosorbide nitrate over a period of at least 24 hours.

3. A multiphase tablet according to claim 1 or 2, wherein the first phase contains 40 to 90% by weight of isosorbide dinitrate or mononitrate and 10 to 60% by weight of hydrophobic and/or lipophilic, non-swelling, fusible carrier or binder.

4. A multiphase tablet according to claims 1, 2 or 3, wherein the active ingredient is isosorbide-5-mononitrate (I-5-MN).

5. A multiphase tablet according to claim 4 wherein the said first phase contains 45 to 70% wt of I-5-MN.

6. A multiphase tablet according to any one of the preceding claims, wherein, the isosorbide nitrate is I-5-MN and when tested according to Ph. Eur. Paddle method in pH 4.5 buffer at 100 rpm, with tablet sinkers and HPLC detection, the amount of I-5-MN released at the intervals specified below expressed as a percentage of the total amount of I-5-MN is as follows:-
| | |
|---|---|
| 1 hour | 15%-50% |
| 2 hours | 25%-65% |
| 4 hours | 40%-85% |
| 8 hours | 60%-100% |
| 12 hours | > 60% |

7. A multiphase tablet according to claim 6, wherein the amounts of I-5-MN released at the intervals specified below are as follows:-
| | |
|---|---|
| 1 hour | 20%-40% |
| 2 hours | 33%-53% |
| 4 hours | 50%-70% |
| 8 hours | 67%-87% |
| 12 hours | 70%-100% |

8. A multiphase tablet according to claim 6, wherein the amount of I-5-MN released at the intervals specified are as follows:-
| | |
|---|---|
| 1 hour | 15%-35% |
| 2 hours | 25%-45% |
| 4 hours | 40%-60% |
| 8 hours | 60%-80% |
| 12 hours | 70%-90% |

9. A multiphase tablet according to claim 6, wherein the amount of I-5-MN released at the intervals specified below are as follows:-
| | |
|---|---|
| 1 hour | 30%-50% |
| 2 hours | 45%-65% |
| 4 hours | 65%-85% |
| 8 hours | > 85% |
| 12 hours | > 90% |

10. A multiphase tablet according to any one of the preceding claims, wherein hydrophobic and/or lipophilic, non-swelling fusible carrier or diluent comprises one or more waxes or oils having a melting point in the range of 35°C to 150°C , preferably 45°C to 90°C.

11. A multiphase tablet according to any one of the preceding claims wherein the wax(es) is/are chosen from hydrogenated vegetable oil and hydrogenated castor oil.

12. A multiphase tablet according to any one of the preceding claims, wherein the second phase contains 0.5% to 20%, preferably 9% to 16% by weight of excipients chosen from anti-tack agents, lubricating and compression aids, with respect to the acetylsalicylic acid or salt thereof in the phase.

13. A multiphase tablet according to claim 12, wherein the second phase contains a compression aid, preferably starch, in an amount of 8% to 13% by weight of the acetylsalicylic and acid or pharmaceutically acceptable salt, and a lubricating agent, preferably a natural or synthetic wax or oil e.g. hydrogenated vegetable oil, in an amount of 0.5% to 6% by weight of the acetylsalicylic acid or pharmaceutically acceptable salt thereof.

14. A multiphase tablet according to any one of the preceding claims wherein when tested by the Ph. Eur. Paddle Method operating at 50 rpm with pH 4.5 buffer as the dissolution medium the second phase is substantially completely disintegrated or the acetylsalicylic acid or salt thereof completely dissolved within 45 minutes.

15. A multiphase tablet according to any one of the preceding claims, wherein the acetylsalicylic acid or pharmaceutically acceptable salt thereof is coated with an enteric coating material or other material e.g. ethyl cellulose, which retards or delays the release thereof.

## Patentansprüche

1. Multiphasentablette für die Behandlung einer Gefäßkrankheit mit einer ersten Phase, die eine therapeutisch wirksame Menge an Isosorbidnitrat, vorzugsweise an Isosorbid-5-Mononitrat, in einer verzögerten Freigabematrix enthält, welche einen hydrophoben und / oder lipophilen, nicht schwellenden, schmelzbaren Träger oder Verdünner aufweist, und mit einer zweiten Phase, die eine therapeutisch wirksame Menge an Acetylsalicylsäure oder einem pharmazeutisch annehmbaren Salz derselben enthält, wobei es keine Barrierenschicht zwischen der ersten und der zweiten Phasen gibt, wobei die zweite Phase so angepasst ist, dass sie sich schnell zersetzt und / oder in der normalen Freisetzungsform vorliegt, und wobei die Tablette, wenn sie durch den Mund eingenommen wird, in der Lage ist, therapeutische Blutspiegel des Isosorbidnitrats über eine Periode von mindestens 12 Stunden zu liefern.

2. Multiphasentablette gemäß Anspruch 1, bei welcher die Tablette in der Lage ist, therapeutische Blutspiegel des Isosorbidnitrats über eine Periode von mindestens 24 Stunden zu liefern.

3. Multiphasentablette gemäß Anspruch 1 oder 2, bei welcher die erste Phase 40 bis 90 Gew.-% des Isosorbiddinitrats oder -mononitrats sowie 10 bis 60 Gew.-% eines hydrophoben und / oder lipophilen, nicht schwellenden, schmelzbaren Trägers oder Bindemittels enthält.

4. Multiphasentablette gemäß den Ansprüchen 1, 2 oder 3, bei welcher das aktive Ingrediens ein Isosorbid-5-mononitrat (I-5-MN) ist

5. Multiphasentablette gemäß Anspruch 4, bei welcher die erste Phase 45 bis 70 Gew.-% an I-5-MN enthält.

6. Multiphasentablette gemäß irgendeinem der vorhergehenden Ansprüche, bei welcher das Isosorbidnitrat aus I-5-MN besteht und, wenn dasselbe gemäß der Ph. Eur. Paddle Methode in einer Pufferlösung mit dem pH-Wert von 4,5 bei 100 Umdrehungen pro Minute (UpM) mit Tablettensetzungsmitteln und mit einem HPLC Nachweis getestet wird, die Menge an I-5-MN, welche in den unten spezifizierten Zeitabständen freigegeben wird und als ein Prozentsatz der gesamten Menge an I-5-MN ausgedrückt wird, sich wie folgt ergibt:
| | |
|---|---|
| 1 Stunde | 15% - 50% |
| 2 Stunden | 25% - 65% |
| 4 Stunden | 40% - 85% |
| 8 Stunden | 60% - 100% |
| 12 Stunden | > 60% |

7. Multiphasentablette gemäß Anspruch 6, bei welcher die Mengen an I-5-MN, welche in den unten spezifizierten Zeitabständen freigegeben worden sind, wie folgt sind:
| | |
|---|---|
| 1 Stunde | 20% - 40% |
| 2 Stunden | 33% - 53% |
| 4 Stunden | 50% - 70% |
| 8 Stunden | 67% - 87% |
| 12 Stunden | 70% - 100% |

8. Multiphasentablette gemäß Anspruch 6, bei welcher der Menge an I-5-MN, welcher in den spezifizierten Zeitabständen freigegeben worden ist, wie folgt ist:
| | |
|---|---|
| 1 Stunde | 15% - 35% |
| 2 Stunden | 25% - 45% |
| 4 Stunden | 40% - 60% |
| 8 Stunden | 60% - 80% |
| 12 Stunden | 70% - 90% |

9. Multiphasentablette gemäß Anspruch 6, bei welcher der Menge an I-5-MN, welcher in den unten spezifizierten Zeitabständen freigegeben worden ist, wie folgt ist:
| | |
|---|---|
| 1 Stunde | 30% - 50% |
| 2 Stunden | 45% - 65% |
| 4 Stunden | 65% - 85% |
| 8 Stunden | > 85% |
| 12 Stunden | > 90% |

10. Multiphasentablette gemäß irgendeinem der vorhergehenden Ansprüche, bei welcher ein hydrophober und / oder lipophiler, nicht schwellender, schmelzbarer Träger oder Verdünner ein Wachs oder Öl oder mehrere davon umfasst, welche einen Schmelzpunkt in dem Bereich von 35°C bis 150 °C, vorzugsweise von 45 °C bis 90 °C, aufweisen.

11. Multiphasentablette gemäß irgendeinem der vorhergehenden Ansprüche, bei welcher das bzw. die Wachse ausgewählt werden aus hydriertem Pflanzenöl und hydriertem Rizinusöl.

12. Multiphasentablette gemäß irgendeinem der vorhergehenden Ansprüche, bei welcher die zweite Phase 0,5 bis 20 Gew.-%, vorzugsweise 9 bis 16 Gew.-%, der Arzneistoffträger enthält, welche ausgewählt werden unter Antiklebrigkeitszusatzstoffen, unter Schmier- und Kompressionshilfsstoffen, unter Berücksichtigung der Acetylsalicylsäure oder der Salze derselben in der Phase.

13. Multiphasentablette gemäß Anspruch 12, bei welcher die zweite Phase einen Kompressionshilfsstoff enthält, vorzugsweise Stärke, in einer Menge von 8 bis 13 Gew.-% der Acetylsalicylsäure und / oder des pharmazeutisch annehmbaren Salzes derselben, und ein Schmiermittel, vorzugsweise ein natürliches oder synthetisches Wachs oder Öl, z.B. ein hydriertes Pflanzenöl, in einer Menge von 0,5 bis 6 Gew.-% der Acetylsalicylsäure oder eines pharmazeutisch annehmbaren Salzes derselben.

14. Multiphasentablette gemäß irgendeinem der vorhergehenden Ansprüche, bei welcher die zweite Phase, wenn sie gemäß der Ph. Eur. Paddle Methode getestet wird, welche bei 50 Umdrehungen pro Minute (UpM) mit einer Pufferlösung mit dem pH-Wert von 4,5 als Auflösungsmittel durchgeführt wird, dann im Wesentlichen vollständig zersetzt ist oder die Acetylsalicylsäure oder das Salz derselben innerhalb von 45 Minuten vollständig aufgelöst ist.

15. Multiphasentablette gemäß irgendeinem der vorhergehenden Ansprüche, bei welcher die Acetylsalicylsäure und / oder ein pharmazeutisch annehmbares Salz derselben mit einem enterischen Beschichtungsmaterial oder mit einem anderen Material, z.B. Ethylzelluose, beschichtet wird, was die Freisetzung der Acetylsalicylsäure und / oder eines pharmazeutisch annehmbaren Salzes derselben verspätet oder verzögert.

## Revendications

1. Cachet à phases multiples, pour le traitement d'un trouble vasculaire possédant une première phase qui contient une quantité thérapeutiquement efficace de nitrate d'isosorbide, de préférence l'isosorbide-5-mononitrate, dans une matrice à libération contrôlée comprenant un véhicule ou un diluant fusible, hydrophobe et/ou lipophile, non gonflant, et une deuxième phase qui contient une quantité thérapeutiquement efficace d'acide acétylsalicylique ou d'un sel pharmaceutiquement acceptable de celui-ci, aucune couche barrière ne se trouvant entre la première et la deuxième phases, la deuxième phase étant adaptée pour se désintégrer rapidement et/ou étant sous une forme de libération normale, et dans lequel le cachet, lorsqu'il est administré oralement, est capable de donner des concentrations sanguines thérapeutiques du nitrate d'isosorbide sur une période d'au moins 12 heures.

2. Cachet à phases multiples suivant la revendication 1, dans lequel le cachet est capable de donner des concentrations sanguines thérapeutiques du nitrate d'isosorbide sur une période d'au moins 24 heures.

3. Cachet à phases multiples suivant la revendication 1 ou 2, dans lequel la première phase contient de 40 à 90% en poids de dinitrate ou de mononitrate d'isosorbide et de 10 à 60% en poids d'un véhicule ou d'un diluant fusible, hydrophobe et/ou lipophile, non gonflant

4. Cachet à phases multiples suivant la revendication 1, 2 ou 3, dans lequel le principe actif est l'isosorbide-5-mononitrate (I-5-MN).

5. Cachet à phases multiples suivant la revendication 4, dans lequel ladite première phase contient de 45 à 70% en poids de I-5-MN.

6. Cachet à phases multiples suivant l'une quelconque des revendications précédentes, dans lequel le nitrate d'isosorbide est I-5-MN et, lorsqu'il est testé suivant la méthode Ph. Eur. Paddle dans un tampon à pH 4,5 à 100 tpm avec des éléments de tassement pour cachets et une détection HPLC, la quantité de I-5-MN libérée aux intervalles spécifiés ci-dessous exprimée sous forme de pourcentage de la quantité totale de I-5-MN est comme suit:
| | |
|---|---|
| 1 heure | 15% - 50% |
| 2 heures | 25% - 65% |
| 4 heures | 40% - 85% |
| 8 heures | 60% - 100% |
| 12 heures | > 60%. |

7. Cachet à phases multiples suivant la revendication 6, dans lequel les quantités de I-5-MN libérées aux intervalles spécifiés ci-dessous sont comme suit:
| | |
|---|---|
| 1 heure | 20% - 40% |
| 2 heures | 33% - 53% |
| 4 heures | 50% - 70% |
| 8 heures | 67% - 87% |
| 12 heures | 70% - 100%. |

8. Cachet à phases multiples suivant la revendication 6, dans lequel les quantités de I-5-MN libérées aux intervalles spécifiés sont comme suit:
| | |
|---|---|
| 1 heure | 15% - 35% |
| 2 heures | 25% - 45% |
| 4 heures | 40% - 60% |
| 8 heures | 60% - 80% |
| 12 heures | 70% - 90%. |

9. Cachet à phases multiples suivant la revendication 6, dans lequel les quantités de 1-5-MN libérées aux intervalles spécifiés ci-dessous sont comme suit:
| | |
|---|---|
| 1 heure | 30% - 50% |
| 2 heures | 45% - 65% |
| 4 heures | 65% - 85% |
| 8 heures | > 85% |
| 12 heures | > 90%. |

10. Cachet à phases multiples suivant l'une quelconque des revendications précédentes, dans lequel le véhicule ou le diluant fusible, hydrophobe et/ou lipophile, non gonflant comprend une ou plusieurs cires ou huiles présentant un point de fusion dans l'intervalle de 35°C à 150°C, de préférence de 45°C à 90°C.

11. Cachet à phases multiples suivant l'une quelconque des revendications précédentes, dans lequel la ou les cires est/sont choisie(s) parmi une huile végétale hydrogénée et une huile de ricin hydrogénée.

12. Cachet à phases multiples suivant l'une quelconque des revendications précédentes, dans lequel la deuxième phase contient de 0,5% à 20%, de préférence de 9% à 16% en poids de véhicules choisis parmi des agents anti-adhésifs, des auxiliaires de lubrification et de compression, par rapport à l'acide acétylsalicylique ou à un sel de celui-ci dans la phase.

13. Cachet à phases multiples suivant la revendication 12, dans lequel la deuxième phase contient un auxiliaire de compression, de préférence un amidon, dans une quantité de 8% à 13% en poids de l'acide acétylsalicylique ou d'un sel pharmaceutiquement acceptable de celui-ci, et un agent de lubrification, de préférence une cire ou une huile naturelle ou synthétique par ex. une huile végétale hydrogénée, dans une quantité de 0,5% à 6% en poids de l'acide acétylsalicylique ou d'un sel pharmaceutiquement acceptable de celui-ci.

14. Cachet à phases multiples suivant l'une quelconque des revendications précédentes, dans lequel, lorsqu'il est testé par la méthode Ph. Eur. Paddle en opérant à 50 tpm avec un tampon à pH 4,5 en tant que milieu de dissolution, la deuxième phase est substantiellement complètement désintégrée ou l'acide acétylsalicylique ou un sel de celui-ci est dissous complètement en 45 minutes.

15. Cachet à phases multiples suivant l'une quelconque des revendications précédentes, dans lequel l'acide acétylsalicylique ou un sel pharmaceutiquement acceptable de celui-ci est enrobé avec une matière d'enrobage entérique ou une autre matière par ex. une éthylcellulose, qui retarde ou diffère la libération de celui-ci.
